Europäisches Patentamt

European Patent Office

Office européen des brevets

(19)

(11) Veröffentlichungsnummer: **0 022 534**
**B1**

(12) **EUROPÄISCHE PATENTSCHRIFT**

(45) Veröffentlichungstag der Patentschrift:
**09.12.81**

(51) Int. Cl.³: **C 07 C 39/08,** C 07 C 37/74

(21) Anmeldenummer: **80103852.2**

(22) Anmeldetag: **07.07.80**

(54) **Verfahren zur Gewinnung von Brenzkatechin und Hydrochinon.**

(30) Priorität: **14.07.79 DE 2928553**

(43) Veröffentlichungstag der Anmeldung:
**21.01.81 Patentblatt 81/3**

(45) Bekanntmachung des Hinweises auf die Patenterteilung:
**09.12.81 Patentblatt 81/49**

(84) Benannte Vertragsstaaten:
**BE CH DE FR GB IT LI NL**

(56) Entgegenhaltungen:
**INDUSTRIAL & ENGINEERING CHEMISTRY, PROD. RES. DEV., Band 15, Nr. 3, 1976, Washington, J. VARAGNAT «Hydroquinone and Pyrocatechol Production by Direct Oxidation of Phenol», Seiten 212 bis 215**

(73) Patentinhaber: **BAYER AG, Zentralbereich Patente, Marken und Lizenzen, D-5090 Leverkusen 1, Bayerwerk (DE)**

(72) Erfinder: **Jupe, Christoph, Dr., Franz-Peter-Kuerten-Weg 2, D-5000 Köln-Dünnwald (DE)**
Erfinder: **Baumert, Jürgen, Dipl.-Ing., An den Kreutzmorgen 7, D-5000 Köln 60 (DE)**
Erfinder: **Schümmer, Günter, Weissdornweg 1, D-5024 Stommeln (DE)**

## Verfahren zur Gewinnung von Brenzkatechin und Hydrochinon

Die vorliegende Erfindung betrifft ein Verfahren zur Gewinnung von Brenzkatechin und Hydrochinon aus Gemischen, die Phenol, Brenzkatechin, Hydrochinon, hochsiedende Begleitstoffe und gegebenenfalls Substanzen enthalten, die Siedepunkte zwischen Phenol und Brenzkatechin haben.

Brenzkatechin und Hydrochinon sind technisch wichtige organische Feinchemikalien, die sowohl direkt, z.B. in photographischen Entwicklern, als auch als Zwischenprodukte für z.B. Farbstoffe, Polymerisationsinhibitoren, Pharmazeutika und Pflanzenschutzmittel Verwendung finden (siehe Kirk-Othmer, Encyclopedia of Chemical Technology, Second Edition, Vol. 11, Seiten 462 bis 492, insbesondere Seiten 469 und 488 [1966]).

Die Suche nach wirtschaftlichen und einfachen Herstellungsverfahren führte u.a. zu einer Reihe von Phenol-Oxidationsverfahren, die Brenzkatechin und Hydrochinon als Koppelprodukte liefern (siehe z.B. DE-OS 2 658 943, DE-OS 2 410 742, DE-OS 2 364 181, DE-OS 2 658 545, DE-OS 2 332 747, DE-OS 1 593 968, DE-OS 2 633 302, DE-AS 2 064 497, DE-OS 2 150 657, DE-OS 2 167 040, DE-OS 2 341 743, DE-OS 2 407 398, DE-OS 1 543 953 und DE-AS 2 404 114).

Heute werden grössere Mengen an Brenzkatechin und Hydrochinon nach diesen Verfahren hergestellt, wie man den Veröffentlichungen von Jean Varagnat, Ind. Eng. Chem., Prod. Res. Dev., Vol. 15, Nr. 3, Seiten 212–215 (1976) und von P. Maggioni und F. Minisci, La Chimica e l'Industria, Vol. 59, Nr. 4, Seiten 239–242 (1977) entnehmen kann.

Bei allen diesen Verfahren, die auf der Hydroxylierung von Phenol mit peroxidischen Reagenzien beruhen, ist zur Erzielung hoher Selektivitäten ein Phenolüberschuss erforderlich. So verlangt z.B das Verfahren gemäss DE-OS 2 407 398 ein Molverhältnis von Phenol zu Wasserstoffperoxid von mehr als 2:1, bevorzugt zwischen 10 und 3:1. Es werden also höchstens zwischen 10 und 50% des Phenols umgesetzt, entsprechend liegen etwa 90 bis 50% des eingesetzten Phenols als nichtumgesetztes Phenol nach der Reaktion vor. Auch das Verfahren gemäss DE-OS 2 064 497 wird bevorzugt bei einem Molverhältnis von Phenol zu Wasserstoffperoxid von 7:1 oder grösser durchgeführt, so dass ein Phenolumsatz von höchstens 15% resultiert und entsprechend 85% des eingesetzten Phenols nach der Umsetzung noch unverändert als Phenol vorliegen.

Um dieses Verfahren in technischem Massstab wirtschaftlich durchführen zu können, ist daher eine Rückgewinnung des nichtumgesetzten Phenols und sein erneuter Einsatz in die Reaktion vorzunehmen. Die Rückgewinnung des Phenols erfolgt in den genannten technisch praktizierten Verfahren durch mehrstufige Destillation. Da Brenzkatechin und Hydrochinon im allgemeinen nicht gemeinsam verwendet werden können, ist es ausserdem erforderlich, Brenzkatechin und Hydrochinon zu trennen, was ebenfalls im allgemeinen durch Rektifikation erfolgt.

In der obenerwähnten Veröffentlichung von J. Varagnat wird dargelegt, wie in einer Folge von fünf Rektifikationskolonnen das Reaktionsgemisch aus der Umsetzung von Phenol destillativ getrennt wird. Phenol und weitere Hilfs- und Begleitstoffe werden in vier Kolonnen als Kopfprodukte gewonnen und wieder verwendet, während Brenzkatechin und Hydrochinon in einer fünften Kolonne getrennt werden und das Hydrochinon anschliessend einer Umkristallisation unterworfen wird. Die Abtrennung von Hochsiedern erfolgt bei diesem Verfahren als Sumpfabnahme der dritten und der fünften Kolonne. Die Abtrennung der Hochsieder in der dritten Kolonne, bei der im Sumpf nur Hochsieder vorliegen, erfordert, dass dort die Gesamtmenge der zu isolierenden Reaktionsprodukte und der Rest des nichtumgesetzten Phenols vollständig verdampft werden. Da zum Betrieb der Kolonne ein gewisser flüssiger Rücklauf erforderlich ist, der einen erneut zu verdampfenden Produktstrom darstellt, muss für die Verdampfung in der dritten Kolonne insgesamt mehr Energie aufgewendet werden, als für die einmalige Verdampfung der zugeführten verdampfbaren Produkte erforderlich ist. Für diese – teilweise mehrfache – Verdampfung muss eine entsprechende Energiemenge aufgebracht werden, die für die Produkte als thermische Belastung wirkt, zumal für den Verdampfer der dritten Kolonne eine Sumpftemperatur von 230 °C angegeben wird. Dem Schema auf Seite 214 der Veröffentlichung von J. Varagnat kann entnommen werden, dass trotz bereits erfolgter Hochsiederabtrennung in der dritten Kolonne die Qualität des Sumpfproduktes der fünften Kolonne (Hydrochinon) nicht für die Entnahme geeignet ist. Das Sumpfprodukt der fünften Kolonne wird nämlich in die dritte Kolonne zurückgefahren und Hydrochinon wird aus der fünften Kolonne als dampfförmiger Seitenstrom entnommen und einer Kristallisation aus Wasser zugeführt. Die Entfernung von Hochsiedern aus dem Sumpf der dritten Kolonne ist also nicht ausreichend, um Hydrochinon als Sumpfprodukt der fünften Kolonne abnehmen zu können.

Auch gemäss dem von P. Maggioni und F. Minisci beschriebenen Verfahren (siehe oben) wird das Reaktionsgemisch destillativ aufgearbeitet. Nach einer stufenweisen Verdampfung in drei hintereinandergeschalteten getrennten Verdampfern bei jeweils niedrigerem Druck bis hinunter zu 13 mbar erfolgt die restliche Auftrennung des Gemisches in zwei Rektifikationskolonnen. In der ersten Kolonne wird Phenol zur Rückführung und in der zweiten Kolonne werden die Produkte Brenzkatechin und Hydrochinon erhalten. Bei diesem Verfahren erfolgt die Hochsiederabtrennung als Sumpfabnahme des dritten Verdampfers, der bei 13 mbar betrieben wird. Die Aufrechterhaltung eines so niedrigen Druckes erfordert einen nicht geringen technischen Aufwand. Auch in diesem Verfahren müssen zur Hochsieder-Abtrennung nicht nur restliches Phenol, sondern auch die Gesamtmenge an Brenzkatechin und Hydrochinon verdampft werden, was Energieaufwand und thermische Belastung der Produkte mit sich bringt. Aus dem Sumpfprodukt der letzten Kolonne wird Hydrochinon abgetrennt und anschliessend aus Wasser umkristallisiert. Die Entfernung von Hochsiedern aus dem Verdampfer ist auch hier nicht ausreichend, denn das Hydrochinon muss vom Sumpfprodukt der letzten Kolonne abgetrennt und dann umkristallisiert werden.

Es wurde nun ein Verfahren zur Gewinnung von Brenzkatechin und Hydrochinon aus Gemischen, die Phenol, Brenzkatechin, Hydrochinon, hochsiedende Begleitstoffe und gegebenenfalls Substanzen enthalten, die zwischen Phenol und Brenzkatechin sieden, gefunden, das dadurch gekennzeichnet ist, dass man

a) solche Gemische einer ersten Rektifikationskolonne zuführt, in der Phenol und gegebenenfalls vorhandene Substanzen, die zwischen Phenol und Brenzkatechin sieden, als Kopfprodukt erhalten werden,

b) das Sumpfprodukt der ersten Rektifikationskolonne einer zweiten Rektifikationskolonne zuführt, in der Brenzkatechin als Kopfprodukt erhalten wird und

c) das Sumpfprodukt der zweiten Rektifikationskolonne einer Aufkonzentriereinheit zuführt, in der Hydrochinon verdampft wird und aus der hochsiedende Begleitstoffe zusammen mit geringen Mengen von Hydrochinon als Rückstand entfernt werden.

In das erfindungsgemässe Verfahren können unterschiedlich zusammengesetzte Gemische eingesetzt werden, die Phenol, Brenzkatechin, Hydrochinon, hochsiedende Begleitstoffe und gegebenenfalls Substanzen enthalten, die zwischen Phenol und Brenzkatechin sieden. Geeignete Gemische können beispielsweise erhalten werden, wenn man Phenol mit einem peroxidischen Hydroxylierungsmittel, gegebenenfalls in Gegenwart von Wasser und/oder organischen Lösungsmitteln, umsetzt und aus den erhaltenen Reaktionsgemischen die unterhalb von Phenol siedenden Bestandteile und gegebenenfalls gemeinsam mit diesen teilweise das Phenol abtrennt. Hochsiedende Begleitstoffe, d.h. Bestandteile des Gemisches, die einen höheren Siedepunkt als Hydrochinon aufweisen oder nicht unzersetzt destillierbar sind, können in der Hydroxylierungsreaktion und/oder den nachfolgenden Aufarbeitungsstufen gebildet worden sein. Substanzen, die zwischen Phenol und Brenzkatechin sieden, können hochsiedende, in die Hydroxylierungsreaktion eingesetzte Lösungsmittel und/oder in der Hydroxylierungsreaktion oder den nachfolgenden Aufarbeitungsstufen gebildete Nebenprodukte sein. Vorzugsweise enthalten die in das erfindungsgemässe Verfahren eingesetzten Gemische keine Substanzen, die zwischen Phenol und Brenzkatechin sieden.

Besonders geeignet zum Einsatz in das erfindungsgemässe Verfahren sind Gemische, die bei der Umsetzung von Phenol mit wässrigem Wasserstoffperoxid oder mit einer Lösung von Wasserstoffperoxid oder einer Percarbonsäure in einem niedriger als Phenol siedenden organischen Lösungsmittel anfallen, nachdem man das Lösungsmittel und gegebenenfalls die der Percarbonsäure entsprechende Carbonsäure und einen Teil des im Überschuss eingesetzten Phenols entfernt hat.

Beispielsweise können in das erfindungsgemässe Verfahren Gemische eingeführt werden, die 10 bis 85 Gew.-% Phenol, jeweils 5 bis 45 Gew.-% Brenzkatechin und Hydrochinon und 0,5 bis 10 Gew.-% hochsiedende Begleitstoffe enthalten. Besonders bevorzugt werden Gemische eingesetzt, die 40 bis 60 Gew.-% Phenol, jeweils 10 bis 30 Gew.-% Brenzkatechin und Hydrochinon und 1 bis 10 Gew.-% hochsiedende Begleitstoffe enthalten.

Im erfindungsgemässen Verfahren wird die erste Rektifikationskolonne so betrieben, dass das gesamte im Einsatzgemisch vorhandene Phenol, gegebenenfalls zusammen mit Substanzen, die zwischen Phenol und Brenzkatechin sieden, über Kopf geht. Von dem Kopfprodukt wird nach Kondensation ein Teil in flüssiger Form als Rücklauf auf die Kolonne gegeben. Der nach Abtrennung des Rücklaufs verbleibende Teil des Kopfproduktes kann beliebig weiter verwendet werden. Vorzugsweise wird er in die Hydroxylierungsreaktion zurückgeführt, gegebenenfalls nach Abtrennung von Substanzen, die zwischen Phenol und Brenzkatechin sieden.

Die erste Rektifikationskolonne kann beispielsweise bei einem Kopfdruck im Bereich von 5 bis 700 mbar betrieben werden. Vorzugsweise liegt der Kopfdruck im Bereich von 50 bis 500 mbar, besonders bevorzugt im Bereich von 100 bis 300 mbar. Die erste Rektifikationskolonne kann beispielsweise zwischen 15 und 25 theoretische Böden enthalten und bei einem Verhältnis von Rücklauf zu Entnahme von 0,5 bis 1,5 betrieben werden. Vorzugsweise enthält die erste Rektifikationskolonne 16 bis 20 theoretische Böden und wird bei einem Verhältnis von Rücklauf zur Entnahme von 0,7 bis 1,3 betrieben. Im Sumpf der ersten Rektifikationskolonne fällt ein Gemisch an, das Brenzkatechin, Hydrochinon und hochsiedende Begleitstoffe enthält.

Dieses Sumpfprodukt wird in die zweite Rektifikationskolonne eingeführt. Diese wird so betrieben, dass als Kopfprodukt praktisch das gesamte vorhandene Brenzkatechin erhalten wird. Von dem Kopfprodukt wird nach Kondensation ein Teil in flüssiger Form als Rücklauf auf die Kolonne gegeben. Der nach Abtrennung des Rücklaufs verbleibende Teil ist das erfindungsgemäss gewonnene Brenzkatechin. Dieses kann auch in fester Form, beispielsweise über eine Schuppenwalze, abgezogen werden.

Die zweite Rektifikationskolonne kann beispielsweise bei einem Kopfdruck im Bereich von 0,02 bis 0,2 bar betrieben werden. Vorzugsweise liegt der Kopfdruck im Bereich 30 bis 100 mbar, ganz besonders bevorzugt ist ein Kopfdruck von 40 bis 70 mbar. Die zweite Rektifikationskolonne kann beispielsweise zwischen 12 und 16 theoretische Böden enthalten und bei einem Verhältnis von Rücklauf zur Entnahme von 1,8 bis 2,5 betrieben werden. Vorzugsweise enthält die zweite Rektifikationskolonne 13 bis 15 theoretische Böden und wird bei einem Verhältnis von Rücklauf zu Entnahme von 2,0 bis 2,2 betrieben. Am Sumpf der zweiten Rektifikationskolonne fällt ein Gemisch an, das Hydrochinon und hochsiedende Begleitstoffe enthält.

Dieses Sumpfprodukt wird einer Aufkonzentriereinheit zugeführt. In der Aufkonzentriereinheit wird die Hauptmenge des vorhandenen Hydrochinons, beispielsweise über 95 Gew.-%, verdampft. Das so gewonnene Hydrochinon kann in vielen Fällen, so wie es, gegebenenfalls nach einer Kondensation, anfällt, verwendet werden. Wenn besondere Anforderungen an seine Reinheit gestellt werden, kann es, z.B. durch eine übliche Umkristallisation, zusätzlich gereinigt werden.

Die Aufkonzentriereinheit wird so betrieben, dass das Sumpfprodukt neben den hochsiedenden Begleitstoffen noch geringe Mengen Hydrochinon enthält. Beispielsweise kann das Sumpfprodukt 0,1 bis 3 Gew.-% Hydrochinon enthalten. Das Sumpfprodukt kann im allgemeinen in flüssiger Form abgetrennt werden. Es wird normalerweise verworfen.

Die Aufkonzentriereinheit kann beispielsweise bei einem Druck von 30 bis 200 mbar betrieben werden. Vorzugsweise arbeitet man bei 35 bis 150 mbar, besonders bevorzugt bei 40 bis 100 mbar.

Die Aufkonzentriereinheit kann auch so betrieben werden, dass man das Sumpfprodukt aus der zweiten Rektifikationskolonne erstarren lässt, in fester Form der Aufkonzentriereinheit zuführt und darin Hydrochinon durch Sublimation abtrennt. In diesem Fall müssen Druck und Temperatur in der Aufkonzentriereinheit so gewählt werden, dass eine Verdampfung aus der festen Phase möglich ist.

Die Durchführung der für die erfindungsgemässen Verfahrensschritte notwendigen Verdampfungen kann im allgemeinen in allen technisch üblichen Verdampfungsapparaturen erfolgen. Beispielsweise sind Umlaufverdampfer oder Fallfilmverdampfer geeignet. Für die im letzten Schritt durchzuführende Verdampfung des Hydrochinons sind Dünnschichtverdampfer und Schneckenverdampfer besonders gut geeignet. Als Kolonnen, die in dem erfindungsgemässen Verfahren verwendet werden können, eignen sich alle technisch üblichen Rektifikationskolonnen, beispielsweise Füllkörperkolonnen oder Glokkenbodenkolonnen. Auch Siebbodenkolonnen oder Kolonnen mit Stahl- oder Keramikpackungen sind gut geeignet.

Für die im erfindungsgemässen Verfahren eingesetzten Apparate sind alle technischen Werkstoffe geeignet, die im Temperaturbereich von 0 bis 250°C gegen die vorhandenen Stoffe beständig sind. Besonders gut geeignet sind Glas, Titan, emaillierte Stähle oder Edelstähle mit hohem Chrom- und Nickel-Gehalt von jeweils beispielsweise zwischen 10 und 30 Gew.-%.

Die Temperaturen, bei denen die einzelnen Verfahrensschritte im allgemeinen durchgeführt werden, sind einerseits durch die Siedepunkte der jeweils zu verdampfenden Substanz(en) bei den angegebenen Drücken und anderseits durch die hohen Temperaturen zu beobachtende Zersetzung der jeweiligen Sumpfphase begrenzt. Die Zersetzung der Sumpfphasen beginnt bei etwa 230°C und ist im allgemeinen ab 250°C stark ausgeprägt. Im allgemeinen wird deshalb bei Temperaturen unterhalb 230°C gearbeitet. Vorzugsweise wird bei Temperaturen unterhalb 215°C gearbeitet, insbesondere an Stellen mit längeren Produkt-Verweilzeiten. Bei kurzen Verweilzeiten können auch Temperaturen im Bereich von 230 bis 250°C angewendet werden.

Mit dem erfindungsgemässen Verfahren gelingt es auf einfachere Weise als bisher, die Produkte Brenzkatechin und Hydrochinon untereinander und von Phenol und hochsiedenden Begleitstoffen abzutrennen. Gegenüber den bekannten Gewinnungsverfahren für Brenzkatechin und Hydrochinon erfordert das erfindungsgemässe Verfahren wesentlich weniger Energie, da Brenzkatechin und Hydrochinon einmal weniger verdampft werden. Ein besonderer Vorteil des erfindungsgemässen Verfahrens besteht darin, dass die hochsiedenden Stoffe nur an einer Stelle isoliert und abgetrennt werden müssen, nämlich als Sumpfprodukt der Aufkonzentriereinheit. Ein weiterer Vorteil des erfindungsgemässen Verfahrens ist es, dass eine schonende Verdampfung der Produkte Brenzkatechin und Hydrochinon gewährleistet ist. In allen Verdampfern, d.h. an den Stellen mit grösstem Wärmeübergang, sind stets Begleitstoffe anwesend, die einen Teil der Wärme übertragen und so die thermische Belastung der Produkte gering halten. Das erfindungsgemässe Verfahren kann bei Drücken durchgeführt werden, die mit technisch üblichen, nicht besonders aufwendigen Mitteln zu erreichen sind.

Es ist als ausgesprochen überraschend anzusehen, dass mit dem erfindungsgemässen Verfahren Brenzkatechin und Hydrochinon von ausreichender Reinheit auf so einfache Weise gewonnen werden können. Aufgrund der bisher bekannten Verfahren und allgemein üblicher verfahrenstechnischer Überlegungen bestand ein Vorurteil dagegen, die Abtrennung der hochsiedenden Begleitstoffe ausschliesslich erst am Ende des Gesamtverfahrens vorzunehmen. Es war zu erwarten, dass beim Führen dieser hochsiedenden Begleitstoffe durch zwei Rektifikationskolonnen und eine Aufkonzentriereinheit Verfestigungen, Ablagerungen und/oder Verstopfungen auftreten und damit insbesondere eine kontinuierliche Durchführung des Gesamtverfahrens mit erheblichen Problemen verbunden ist. Überraschenderweise bleiben die hochsiedenden Begleitstoffe in den Sumpfphasen im erfindungsgemässen Verfahren jedoch flüssig und damit ohne besondere Probleme handhabbar.

Beispiel

Ein Gemisch aus 51,1 Gew.-% Phenol, 25,2 Gew.-% Brenzkatechin, 17,7 Gew.-% Hydrochinon und 5,9 Gew.-% an hochsiedenden Begleitstoffen wurde kontinuierlich einer ersten Rektifikationskolonne aus Glas zugeführt, die einen Innendurchmesser von 50 mm, eine Füllung aus 4 × 4 mm Glas-Ringen und eine Länge von 1200 mm im Verstärker- und 1500 mm im Abtreiber-Teil hatte. Die Kolonne war im Verstärkerteil auf je 500 mm und im Abtriebsteil auf je 750 mm mit einer adiabaten Beheizung und über die Gesamtlänge mit einer Schutzheizung versehen.

Bei einem Kopfdruck von 136 mbar, einem Rücklaufverhältnis von 1 und einer Sumpftemperatur von 201 °C wurde Phenol als Kopfprodukt erhalten.

Das Sumpfprodukt enthielt 52 Gew.-% Brenzkatechin, 36 Gew.-% Hydrochinon und 12 Gew.-% hochsiedende Begleitstoffe.

Das aus der ersten Rektifikationskolonne als Sumpfgemisch erhaltene Produkt-Gemisch wurde kontinuierlich einer zweiten Rektifikationskolonne aus Glas mit folgenden Abmessungen zugeführt:

Durchmesser: 50 mm; Füllkörper: 4 × 4 mm Glas-Ringe; Verstärkerteil: 2000 mm Länge; Abtreiberteil: 1000 mm Länge.

Die Kolonne war ebenfalls mit einer Schutzheizung und über die gesamte Länge auf je 500 mm mit einem adiabatischen Heizmantel versehen.

Bei einem Kopfdruck von 42 mbar und einem Verhältnis von Rücklauf zu Entnahme von 2 wurde als Kopfprodukt reines Brenzkatechin abgezogen, im Sumpf fiel bei 206 °C ein Gemisch aus 75 Gew.-% Hydrochinon und 25 Gew.-% Begleitstoffen an.

Das so erhaltene Sumpfprodukt wurde kontinuierlich einem Dünnschichtverdampfer zugeführt, in dem bei 40 mbar und 186 °C Hydrochinon abdestilliert und ein flüssiger Sumpf anfiel, der 1 Gew.-% Hydrochinon enthielt.

**Patentansprüche**

1. Verfahren zur Gewinnung von Brenzkatechin und Hydrochinon aus Gemischen, die Phenol, Brenzkatechin, Hydrochinon, hochsiedende Begleitstoffe und gegebenenfalls Substanzen enthalten, die zwischen Phenol und Brenzkatechin sieden, dadurch gekennzeichnet, dass man

a) solche Gemische einer ersten Rektifikationskolonne zuführt, in der Phenol und gegebenenfalls vorhandene Substanzen, die zwischen Phenol und Brenzkatechin sieden, als Kopfprodukt erhalten werden,

b) das Sumpfprodukt der ersten Rektifikationskolonne einer zweiten Rektifikationskolonne zuführt, in der Brenzkatechin als Kopfprodukt erhalten wird und

c) das Sumpfprodukt der zweiten Rektifikationskolonne einer Aufkonzentriereinheit zuführt, in der Hydrochinon verdampft wird und aus der hochsiedende Begleitstoffe zusammen mit geringen Mengen von Hydrochinon als Rückstand entfernt werden.

2. Verfahren nach Anspruch 1, dadurch gekennzeichnet, dass man ein Gemisch einsetzt, das erhalten worden ist, indem man Phenol mit einem peroxidischen Hydroxylierungsmittel gegebenenfalls in Gegenwart von Wasser und/oder organischen Lösungsmitteln umgesetzt und aus den erhaltenen Reaktionsgemischen die unterhalb von Phenol siedenden Bestandteile, gegebenenfalls gemeinsam mit einem Teil des Phenols, abgetrennt hat.

3. Verfahren nach Ansprüchen 1 und 2, dadurch gekennzeichnet, dass man ein Gemisch einsetzt, das 10 bis 85 Gew.-% Phenol, jeweils 5 bis 45 Gew.-% Brenzkatechin und Hydrochinon und 0,5 bis 10 Gew.-% hochsiedende Begleitstoffe enthält.

4. Verfahren nach Ansprüchen 1 bis 3, dadurch gekennzeichnet, dass die erste Rektifikationskolonne bei einem Kopfdruck im Bereich von 5 bis 700 mbar betrieben wird.

5. Verfahren nach Ansprüchen 1 bis 4, dadurch gekennzeichnet, dass die erste Rektifikationskolonne zwischen 15 und 25 theoretische Böden enthält und bei einem Verhältnis von Rücklauf zu Entnahme von 0,5 bis 1 betrieben wird.

6. Verfahren nach Ansprüchen 1 bis 5, dadurch gekennzeichnet, dass die zweite Rektifikationskolonne bei einem Kopfdruck im Bereich von 0,02 bis 0,2 bar betrieben wird.

7. Verfahren nach Ansprüchen 1 bis 6, dadurch gekennzeichnet, dass die zweite Rektifikationskolonne zwischen 12 und 16 theoretische Böden enthält und bei einem Verhältnis von Rücklauf zu Entnahme von 1,8 bis 2,5 betrieben wird.

8. Verfahren nach Ansprüchen 1 bis 7, dadurch gekennzeichnet, dass die Aufkonzentriereinheit bei einem Druck von 30 bis 200 mbar betrieben wird.

9. Verfahren nach Ansprüchen 1 bis 8, dadurch gekennzeichnet, dass die Aufkonzentriereinheit so betrieben wird, dass das Sumpfprodukt 0,1 bis 3 Gew.-% Hydrochinon enthält.

10. Verfahren nach Ansprüchen 1 bis 9, dadurch gekennzeichnet, dass die einzelnen Verfahrensschritte bei Temperaturen unterhalb 230 °C durchgeführt werden.

**Claims**

1. Process for the isolation of pyrocatechol and hydroquinone from mixtures which contain phenol, pyrocatechol, hydroquinone, high-boiling concomitant substances and, if appropriate, substances with boiling points between those of phenol and pyrocatechol, characterized in that a) such mixtures are fed to a first rectification column in which the phenol and any substances present which have boiling points between those of phenol and pyrocatechol are obtained as the top product, b) the bottom product of the first rectification column is fed to a second rectification column in which the pyrocatechol is obtained as the top product and c) the bottom product from the second rectification column is fed to a concentrating unit in which hydroquinone is evaporated and from which high-boiling concomitant substances are removed as the residue, together with small amounts of hydroquinone.

2. Process according to claim 1, characterized in that a mixture which has been obtained by reacting phenol with a peroxidic hydroxylating agent, if appropriate in the presence of water and/or organic solvents, and by separating off the constituents with boiling points below that of phenol, if appropriate together with some of the phenol, from the resulting reaction mixtures is employed.

3. Process according to claims 1 and 2, characterized in that a mixture which contains 10 to 85% by weight of phenol, 5 to 45% by weight each of pyrocatechol and hydroquinone and 0,5 to 10% by weight of high-boiling concomitant substances is employed.

4. Process according to claims 1 to 3, characterized in that the first rectification column is operated under an overhead pressure in the range from 5 to 700 mbars.

5. Process according to claims 1 to 4, characterized in that the first rectification column contains between 15 and 25 theoretical plates and is operated at a ratio of reflux to removal of 0,5 to 1.

6. Process according to claims 1 to 5, characterized in that the second rectification column is operated under an overhead pressure in the range from 0,02 to 0,2 bar.

7. Process according to claims 1 to 6, characterized in that the second rectification column contains between 12 and 16 theoretical plates and is operated at a ratio of reflux to removal of 1,8 to 2,5.

8. Process according to claims 1 to 7, characterized in that the concentrating unit is operated under a pressure of 30 to 200 mbars.

9. Process according to claims 1 to 8, characterized in that the concentrating unit is operated such that the bottom product contains 0,1 to 3% by weight of hydroquinone.

10. Process according to claims 1 to 9, characterized in that the individual process steps are carried out at temperatures below 230°C.

**Revendications**

1. Procédé de récupération de pyrocatéchine et d'hydroquinone à partir de mélanges qui contiennent du phénol, de la pyrocatéchine, de l'hydroquinone, des substances d'accompagnement à point d'ébullition élevé et éventuellement des substances qui bouillent entre le phénol et la pyrocatéchine, caractérisé en ce qui:

a) on alimente ces mélanges dans une première colonne de rectification dans laquelle le phénol et éventuellement les substances présentes qui bouillent entre le phénol et la pyrocatéchine sont obtenus comme produit de tête,

b) on alimente le produit de queue de la première colonne de rectification dans une seconde colonne de rectification dans laquelle la pyrocatéchine est obtenue comme produit de tête et

c) on alimente le produit de queue de la seconde colonne de rectification dans une unité de concentration dans laquelle l'hydroquinone est vaporisée et à partir de laquelle les substances d'accompagnement à point d'ébullition élevé sont écartées en même résidu.

2. Procédé selon la revendication 1, caractérisé en ce qu'on utilise un mélange qui est obtenu en faisant réagir du phénol un agent d'hydroxylation peroxydé éventuellement en présence d'eau et/ou de solvants organiques, et en ce qu'à partir des mélanges de réaction obtenus on sépare les constituants bouillant au-dessous du phénol, éventuellement conjointement avec une partie du phénol.

3. Procédé selon les revendications 1 et 2, caractérisé en ce qu'on utilise un mélange qui contient 10 à 85% en poids de phénol, 5 à 45% en poids chacun de pyrocatéchine et d'hydroquinone et 0,5 à 10% en poids de substances d'accompagnement à point d'ébullition levé.

4. Procédé selon les revendications 1 à 3, caractérisé en ce que la première colonne de rectification est en fonctionnement à une pression de tête dans l'intervalle de 5 à 700 mbars.

5. Procédé selon les revendications 1 à 4, caractérisé en ce que la première colonne de rectification contient entre 15 et 25 plateaux théoriques et est en fonctionnement à un rapport reflux/prélèvement de 0,5 à 1.

6. Procédé selon les revendications 1 à 5, caractérisé en ce que la seconde colonne de rectification est en fonctionnement à une pression de tête dans l'intervalle de 0,02 à 0,2 bar.

7. Procédé selon les revendications 1 à 6, caractérisé en ce que la seconde colonne de rectification contient entre 12 et 16 plateaux théoriques et est en fonctionnement à un rapport reflux/prélèvement de 1,8 à 2,5.

8. Procédé selon les revendications 1 à 7, caractérisé en ce que l'unité de concentration est en fonctionnement à une pression de 30 à 200 mbars.

9. Procédé selon les revendications 1 à 8, caractérisé en ce que l'unité de concentration est mise en fonction de telle sorte que le produit de queue contienne 0,1 à 3% en poids d'hydroquinone.

10. Procédé selon les revendications 1 à 9, caractérisé en ce que les divers stades du procédé sont exécutés à des températures inférieures à 230°C.